# EUROPEAN PATENT SPECIFICATION

(11) **EP 3 378 947 B1**
(45) Date of publication and mention of the grant of the patent: **24.01.2024**
(21) Application number: 15908514.1
(22) Date of filing: 16.11.2015
(51) Int. Cl.: C12Q 1/68

(54) **USE OF ED-B PROTEIN IN DIAGNOSIS OF TISSUE HYPERPLASIA**
VERWENDUNG VON ED-B-PROTEIN BEI DER DIAGNOSE VON GEWEBEHYPERPLASIE
UTILISATION DE PROTÉINE ED-B DANS LE DIAGNOSTIC D'UNE HYPERPLASIE TISSULAIRE

(43) Date of publication of application: 26.09.2018
(73) Proprietor: Hefei Lifeon Pharmaceutical Co. Ltd., Hefei, Anhui 230088 (CN)
(72) Inventor: JI, Junqiu, Hefei Anhui 230088 (CN); ZHANG, Mei, Hefei Anhui 230088 (CN); GAO, Meihua, Hefei Anhui 230088 (CN); CHEN, Jun, Hefei Anhui 230088 (CN)
(74) Representative: Nederlandsch Octrooibureau
(86) International application number: PCT/CN2015/094727
(87) International publication number: WO 2017/084017

(56) References cited:
- WO-A1-2007/128557
- WO-A2-01/62298
- WO-A2-01/83816
- CN-A- 1 612 895
- CN-A- 101 356 271
- CN-A- 101 801 420
- CN-A- 102 015 768
- S. SAUER ET AL: "Expression of the oncofetal ED-B-containing fibronectin isoform in hematologic tumors enables ED-B-targeted 131I-L19SIP radioimmunotherapy in Hodgkin lymphoma patients", BLOOD, vol. 113, no. 10, 5 March 2009 (2009-03-05), pages 2265-2274, XP055590760, US ISSN: 0006-4971, DOI: 10.1182/blood-2008-06-160416
- Marta Midulla ET AL: "Source of Oncofetal ED-B-containing Fibronectin: Implications of Production by Both Tumor and Endothelial Cells 1", CANCER RESEARCH United Kingdom; Antisoma plc, 1 January 2000 (2000-01-01), pages 164-169, XP055305964, Retrieved from the Internet: URL:http://cancerres.aacrjournals.org/cont ent/canres/60/1/164.full.pdf

## Description

### Field of the Invention

The invention relates to a novel method for detecting tissue hyperplasia, and belongs to the technical fields of medicine and biotechnology. In particular, the invention relates to an ED-B domain containing protein or a fragment thereof for use in tumor diagnosis or screening, and as a marker of tumor and tissue hyperplasia diseases, an ED-B domain can be used in tumor diagnosis, tumor prognosis evaluation, and tumor early prevention and screening in general population and the like.

### Background of the Invention

Tumors are now the second leading cause of death from non-infectious diseases in the world. Tumor researchers believe that tumors can be prevented, early tumors can be cured, and the quality of life of the patients with advanced tumors can also be improved through standardized treatment. However, despite the great progress made in modern medical technology, the prognosis of cancer treatment is poor for patients with advanced tumors, especially after the tumors have spread. In clinical practice, early detection and early treatment of tumors are critical, which require the support of effective diagnostic methods and techniques.

Tumor diagnosis methods include: 1. medical history and physical examination; 2. endoscopy; 3. imageological examination such as X-ray examination, ultrasound examination and radionuclide scanning; 4. pathological examination such as cytologic examination and biopsy; 5. laboratory enzymological or immunological examination, etc. Laboratory immunological examination of tumor markers or related substances can improve the sensitivity and specificity of early detection of tumors and facilitate the discovery and treatment of tumors. As some of the tumor markers are present in the blood of patient, these tumor blood markers provide convenience for early diagnosis of tumors and screening in the general population; the collection of a small amount of blood is safe and convenient; immunological examination does not require large-scale instruments and is easy to popularize; an obtained sample can be detected and analyzed elsewhere; and compared with endoscopy and imageological examination, patients receiving an immunological examination of blood markers have good compliance. Therefore, the discovery and use of tumor markers or tumor-related substances are effective methods to improve the efficiency of tumor diagnosis.

The combination of monoclonal antibodies specific for tumor markers and sensitive immunological detection technologies (ELISA, RIA, IRMA, CLIA, IFA, TRFIA, etc.) produces a series of methods for detecting tumor markers which are applied in clinical practice, being a powerful tool for clinical diagnosis of tumors. The diagnostic application of tumor markers are becoming more and more widespread, such as: tumor survey and screening in population; diagnosis/confirmation and assessing progression stages of tumors; analyzing the biological characteristics of diseases and conditions; therapeutic effect monitoring and prognosis; judgment of secondary tumors and primary tumors; and combined application of multiple tumor markers to improve detection efficiency, etc.

Tumor markers refer to specific molecules produced by tumor cells or tissues during the occurrence or development of tumors. These molecules are few or even difficult to be detected in the body of healthy people, but their content may increase significantly or change remarkably along with the occurrence or development of tumors. These molecules can be proteins, polypeptides, nucleic acids, small molecule compounds, and the like. Nearly a hundred clinically significant tumor markers have been discovered so far, among which there are a dozen frequently used including CEA, AFP, CYFRA21-1, CA125 and CA15-3. An ideal tumor marker should have high sensitivity and specificity and certain organ specificity, be related to the size, growth or progress of tumors as well as prognosis, and be helpful for monitoring of tumor recurrence. Currently found tumor markers only have several of the above characteristics in most cases and are far from the ideal, so further research is needed.

Fibronectin (FN) is a multifunctional glycoprotein expressed by epithelial cells, endothelial cells, fibroblasts, hepatocytes, decidual cells, extravillous trophoblast cells, etc. There are two types of FN: plasma FN (pFN) present in plasma and various body fluids in a soluble form, involved in coagulation and other functions; and cellular FN (cFN) present in extracellular matrix, between cells, and on the surface of certain cells in the form of insoluble fibers, involved in cell adhesion, deformation and distribution, as well as the formation of blood vessels, etc. The use of FN has become increasingly widespread. FN has been used in repair and healing of wounds, emergency aid and treatment of sepsis and shock, and plays an important role in the pathogenesis of hematological diseases, cardiovascular and cerebrovascular diseases, diabetes, etc.

The gene of FN is about 75 kb in length, containing about 50 exons, and having a relative molecular mass of about 250 kDa. FN is mainly composed of three types, type I, type II and type III, of homologous repetitive spherical domain units, and different domains are linked through a peptide chain which is sensitive to trypsin. Each FN subunit has a site for high-affinity binding to collagen, cell surface receptors, fibrin and sulfated proteoglycan. Plasma FN is a dimer consisting of two similar subunits, two disulfide bridges are formed at the C-terminus of each single strand for crosslinking, and the entire molecule is V-shaped. Cellular FN is a multimer, and its FN subunits from different sources are similar but not identical in structure.

The concentration of plasma FN (pFN) in the blood of healthy people is 277-513 mg/L, and the increase in the concentration of pFN is observed in acute and chronic hepatitis, fatty liver, liver cirrhosis, obstructive jaundice, cerebrovascular disease, late pregnancy, pancreatic cancer, lung cancer, cancerous ascites, extensive metastasis of adenocarcinoma, etc.; and the decrease in the concentration of pFN is observed in acute leukemia, fulminant hepatic failure, burns, trauma, shock, bacterial or viral infections, acute respiratory distress syndrome, diabetic ketoacidosis, uremia, acute circulatory failure, menopausal women, and some patients with poor tolerance to chemotherapy.

FNs are expression products of the same gene, but are different in the splicing of RNA after transcription, generating different mRNAs and different FN isomers, and also different in post-translational modification. Thus, there are FN(B+) (or B-FN or FN(ED-B)) containing ED-B domains and FN(B-) containing no ED-B domain during the expression of FN genes. Both forms are thought to play an important role during the individual development.

ED-B (Extradomain B) is a complete domain containing 91 amino acids, encoded by a single exon and contained in the type III repetitive sequence of FN. FN(B+) is rarely expressed in normal tissue of adults, but its expression is increased in rapidly hyperplastic tissues such as wound healing tissue and tumor tissue, and especially it is highly expressed again during tumor growth, thus called carcinoembryonic gene, e.g. ED-B domain containing FN(B+) is highly expressed in cells such as gastric cancer cells, colorectal cancer cells, lung cancer cells and breast cancer cells. Almost all human solid tumors have high FN(B+) expression in their primary lesions and metastatic sites. FN(B+) may play an important role in tumor invasion and metastasis. Based on the above facts, FN(B+) is used as a marker of tumor neovascularization (Carnemolla, Balza et al. (1989). J Cell Biol. 108: 1139-1148). However, the study on human ED-B domain or ED-B domain containing fibronectin FN(ED-B) is not sufficient, and its biological characteristics need to be further explored.

The detection of ED-B in blood samples or tissue homogenates is rarely reported, and corresponding detection methods need to be further developed. At present, there is no report on the presence of ED-B in blood. The current available information is that FN in blood does not contain ED-B domain (Zardi, Carnemolla et al. (1987). EMBO J. 6: 2337-2342). For example, ED-B was not detected by detecting pFN through enzyme-linked immunosorbent assay (ELISA) (Carnemolla, Neri et al. (1996). Int J Cancer. 68: 397-405). It is also indicated that ED-B is negative in pFN by Western Blot assay (Viti, Tarli et al. (1999). Cancer Res. 59: 347-352.). Up to now, there is no report that ED-B is detected in blood.

### Summary of the Invention

In one aspect, the invention provides a method for detecting tissue hyperplasia in a mammal, such as human being, diagnosing the presence or risk of solid tumors, or prognosing solid tumors, comprising the steps of detecting the level of ED-B protein in a blood sample of the mammal, and comparing with the level of the ED-B protein in a healthy control blood sample, wherein the increased level of the ED-B protein in the detected sample is indicative of the presence of tissue hyperplasia or the presence or risk of solid tumors in the mammal, or poor prognosis, wherein the level of the ED-B protein in the biological sample is detected by means of a binding agent specifically detecting the ED-B protein, wherein the ED-B protein comprises: (a) SEQ ID NO:1, or (b) a sequence having at least 80%, 85%, 90%, 95%, 96%, 97%, 98%, 99% or more sequence identity with the sequence of (a), and wherein the binding agent specifically detecting the ED-B protein is an antibody to the ED-B protein, such as a monoclonal or polyclonal antibody.

The method can be used to diagnose the presence or risk of a tumor, perform tumor prognosis, diagnose the occurrence or progression of a tumor, perform tumor screening or monitor the efficacy of oncotherapy.

In one aspect, the invention provides use of a binding agent specifically detecting ED-B protein for the detection of ED-B protein in a blood sample, of a mammal such as human being.

In one aspect, the invention provides a kit for detecting ED-B protein in a blood sample, of a mammal such as human being, wherein the kit comprises: i) the binding agent specifically detecting the ED-B protein in the blood sample, of the mammal; and, ii) an anticoagulant, wherein the level of the ED-B protein in the biological sample is detected by means of a binding agent specifically detecting the ED-B protein, wherein the ED-B protein comprises: (a) SEQ ID NO:1, or (b) a sequence having at least 80%, 85%, 90%, 95%, 96%, 97%, 98%, 99% or more sequence identity with the sequence of (a), and wherein the binding agent specifically detecting the ED-B protein is an antibody to the ED-B protein, such as a monoclonal or polyclonal antibody.

### Brief Description of the Drawings

FIG. 1: SDS-PAGE detection of pED-B levels in the blood of healthy people and tumor patients. The four lanes from left to right are bovine serum albumin (BSA), ED-B antibody (B5-Fc), tumor pED-B and healthy pED-B, wherein BSA and B5-Fc are used as controls. The level of pED-B in plasma of tumor patients is significantly higher than the level of pED-B in healthy people.
FIG. 2: Western Blot detection of the pED-B protein obtained by the pull down method. The four lanes from left to right are bovine serum albumin (BSA), ED-B antibody (B5-Fc), tumor pED-B and healthy pED-B, wherein both the third and fourth lanes are positive for pED-B and B5-Fc, the second lane is positive for B5-Fc only (approximately 55 kD), and the first lane is negative.
FIG. 3: Results of detecting different types of samples by ED-B detection kit.
FIG. 4A-J: The concentration of pED-B in the blood of healthy people, tumor patients and patients with other diseases.
FIG. 5: Standard curve in a pED-B test using B5 and L19 antibodies.

### Detailed Description of the Invention

Unless otherwise specified, scientific and technical terms used herein should have meanings that are generally known to those skilled in the art. In addition, singular terms shall include the plural, and plural terms shall include the singular unless specifically required. The foregoing techniques and methods are generally performed according to conventional methods well known in the art and described in the references cited in this specification. See, for example, Sambrook et al. Molecular Cloning: A Laboratory Manual (3rd ed., Cold Spring Harbor Laboratory Press, Cold Spring Harbor, N. Y. (2001)).

The purpose of the invention is to provide a novel tissue hyperplasia marker and related detection methods and uses such as human tissue hyperplasia screening and diagnosis. The marker may serve as a tumor blood marker to provide a basis for screening, diagnosis, treatment and prognosis of tumors.

The invention is based on two new discoveries. One is the presence of ED-B protein in blood, including the presence of the ED-B domain in part of plasma fibronectin (pFN) in blood, or the presence of the ED-B domain in hydrolysis fragments of FN. The soluble ED-B protein in blood is abbreviated as pED-B (Plasma ED-B). The other is that the level of the ED-B protein in blood is related to the generation and malignancy of various tissue hyperplasias, such as tumors.

Thus, in one aspect, the invention provides a method for detecting tissue hyperplasia in a mammal, such as human being, diagnosing the presence or risk of solid tumors, or prognosing solid tumors, comprising the steps of detecting ED-B protein in a blood sample of the mammal, and comparing with the level of the ED-B protein in a healthy control blood sample, wherein the increased level of the ED-B protein in the detected sample is indicative of the presence of tissue hyperplasia or the presence or risk of solid tumors in the mammal, or poor prognosis, wherein the level of the ED-B protein in the biological sample is detected by means of a binding agent specifically detecting the ED-B protein, wherein the ED-B protein comprises: (a) SEQ ID NO:1, or (b) a sequence having at least 80%, 85%, 90%, 95%, 96%, 97%, 98%, 99% or more sequence identity with the sequence of (a), and wherein the binding agent specifically detecting the ED-B protein is an antibody to the ED-B protein, such as a monoclonal or polyclonal antibody.

As used herein, "increased level" refers to an increase in the measured value obtained by detecting the sample from a reference value, such as an average or median value observed in a patient who does not have a tumor or a normal person, for example, an increase of at least 5%, at least 10%, at least 20%, at least 30%, at least 40%, at least 50%, at least 60%, at least 70%, at least 80%, at least 90%, at least 100%, at least 2 times, at least 3 times, at least 4 times, at least 5 times, at least 10 times, at least 20 times, at least 30 times, at least 40 times, at least 50 times or more.

In one embodiment, the method according to the invention is used for diagnosing the presence or risk of a disease associated with hyperplasia in the mammal, such as a tumor, wherein the increased level of the ED-B protein in the detected sample is indicative of the presence or risk of the tumor in the mammal.

In one embodiment, the method according to the invention is used for prognosis of a disease associated with hyperplasia in the mammal, such as a tumor, wherein the increased level of the ED-B protein in the detected sample is indicative of poor prognosis of the tumor in the mammal.

Disclosed is that the method may be used for determining the occurrence or progression of a tumor, performing tumor screening or monitoring the efficacy of oncotherapy, or for personalized medication.

As used herein, the term "ED-B protein" refers to a protein containing ED-B amino acid sequence, wherein the ED-B amino acid sequence has the amino acid sequence shown in SEQ ID No. 1 or a sequence having at least 80%, 85%, 90%, 95%, 96%, 97%, 98% and 99% or more sequence identity with SEQ ID No. 1. As used herein, the term "ED-B protein domain" or "ED-B domain" refers to a protein tertiary structure formed by the amino acid sequence of SEQ ID No. 1, and the ED-B domain exists as part of the protein. As used herein, the term "ED-B protein fragment" refers to a protein fragment containing ED-B amino acid sequence formed through denaturation, breakage, degradation and hydrolysis of a protein containing ED-B domain under physical, chemical and biological conditions etc. The ED-B protein is a collective term for the above-mentioned protein, domain and fragment containing ED-B amino acid sequence, and comprises but not limited to a protein containing ED-B domain or a protein containing an ED-B peptide fragment.

As used herein, the term "pED-B" is an abbreviation for ED-B protein derived from blood, such as plasma. pED-B refers to a protein containing ED-B amino acid sequence in blood, and can exist in blood alone in a free state, or as a domain in FN or an FN hydrolysis fragment, or combined with other extracellular proteins in blood. The pED-B protein is a component in blood, but may also be a hydrolyzed fragment derived from cellular FN(B+).

In addition, the ED-B protein according to the invention also includes a protein containing the ED-B amino acid sequence, obtained based on artificially synthesized peptide fragments or random mutations of the amino acid sequence of SEQ ID No. 1, or through genetic recombination. However, since the ED-B protein obtained artificially is not derived directly from an organism, it is not pED-B.

The protein or protein fragment of the invention may be a phosphorylated or glycosylated protein or a denatured protein or polypeptide fragment. In one embodiment, the ED-B protein of the invention consists of the amino acid sequence of SEQ ID No. 1 or a sequence having at least 80%, 85%, 90%, 95%, 96%, 97%, 98% and 99% or more sequence identity with SEQ ID No. 1, or comprises partial continuous sequence of SEQ ID No. 1 or a sequence having at least 80%, 85%, 90%, 95%, 96%, 97%, 98% and 99% or more sequence identity with SEQ ID No. 1. In one embodiment, the ED-B protein or protein fragment of the invention is FN(B+) containing the ED-B domain, and a polymer, a dimer, a monomer, an isomer, a subtype and a hydrolyzed fragment of FN (B+), etc.

Disclosed is also a nucleotide sequence encoding a peptide comprising the amino acid sequence of SEQ ID No. 1 or a sequence having at least 80%, 85%, 90%, 95%, 96%, 97%, 98%, 99% or more sequence identity with SEQ ID No. 1, or consisting of SEQ ID No. 1 or a sequence having at least 80%, 85%, 90%, 95%, 96%, 97%, 98%, 99% or more sequence identity with SEQ ID No. 1. The nucleotide sequence may comprise or consist of the sequence of SEQ ID No.2.

The term "tissue hyperplasia" refers to an increase in the number of cells in organs and tissues, producing neoplasms, tumors or cancers. Tissue hyperplasia can be physiological hyperplasia or pathological hyperplasia, wherein physiological hyperplasia, such as connective tissue hyperplasia, is an important reaction in the process of wound healing and plays an active role in the adaptive response of the body. Pathological hyperplasia is mostly caused by overstimulation of endocrine hormones or local inflammatory factors, such as breast hyperplasia, prostate hyperplasia, endometrial hyperplasia and intestinal mucosal polyps.

As used herein, the terms "polypeptide", "peptide" and "protein" can be used interchangeably, refer to a polymer of amino acid residues, and comprise 9 or more amino acids joined by peptide bond. The polymer may be linear, branched or cyclic, contain naturally occurring amino acids and/or amino acid analogs, and may be interrupted by non-amino acids. Generally, if the amino acid polymer is long, for example more than 50 amino acid residues, it is preferably called as a polypeptide or protein, and if it is 50 amino acids or shorter, it is preferably called as a "peptide".

As used herein, the terms "nucleic acid", "nucleic acid molecule", "polynucleotide" and "nucleotide sequence" may be used interchangeably, to define a polymer of any length, such as polydeoxyribonucleotides (DNA) (for example, cDNA, genomic DNA, plasmids, vectors, viral genomes, isolated DNA, probes, primers and any mixture thereof) or polyribonucleotide (RNA) molecules (for example, mRNA, antisense RNA) or mixed polyribose-polydeoxyribonucleotides. They include single- or double-stranded, linear or circular, natural or synthetic polynucleotides. In addition, polynucleotides can include non-naturally occurring nucleotides, such as methylated nucleotides and nucleotide analogs, and can be interrupted by non-nucleotide components. Nucleotide modification, if any, may be made before or after polymerization.

The ED-B protein may refer to a protein containing the ED-B amino acid sequence or a proteolytic fragment containing the ED-B amino acid sequence or a proteolytic fragment containing only ED-B domain in blood, serum or plasma.

In one embodiment, the tumor according to the invention is a solid tumor. In one embodiment, the solid tumor according to the invention may be a benign tumor or a malignant tumor. In one embodiment, the solid tumor according to the invention is an upper gastrointestinal tumor. The upper gastrointestinal tumor according to the invention includes, but not limited to esophageal cancer, cardia cancer, laryngeal cancer and gastric cancer.

The term "malignant tumor" as used herein refers to a tumor or cancer which not only exhibits uncontrolled abnormal proliferation of the cells of the tumor itself, but also often is lethal and invades adjacent normal tissues and metastasizes to other parts of the body via blood vessels, lymphatic vessels and body cavities. In one embodiment, the tumor according to the invention is a malignant tumor such as cancer or sarcoma, for example, various squamous cell carcinomas, adenocarcinomas and sarcomas. In one embodiment, the tumor according to the invention is selected from the group consisting of esophageal cancer, cardia cancer, laryngeal cancer, head and neck cancer, gastric cancer, liver cancer, biliary tract cancer, gallbladder cancer, colon cancer, duodenal cancer, lung cancer, bladder cancer, cervical cancer, ovarian cancer, endometrial cancer, breast cancer, melanoma, pancreatic cancer, kidney cancer and prostate cancer. In one embodiment, the tumor according to the invention is an upper gastrointestinal cancer, for example, selected from the group consisting of esophageal cancer, cardia cancer, laryngeal cancer and gastric cancer. In one embodiment, the tumor is nasopharyngeal cancer, esophageal cancer, gastric cancer, lung cancer or pancreatic cancer.

The biological sample described herein is a biological sample from a tested animal, which comprises nucleic acids or polypeptides. Advantageously, the following samples may be included: blood, plasma, platelets, saliva, sputum, urine, etc., more generally any tissue, organ, or advantageously a biological body fluid comprising nucleic acids or polypeptides. The sample used for detection in the *in vitro* method of the invention should generally be collected in a clinically acceptable manner, for example in the manner of protecting nucleic acids or proteins. The sample to be tested may be a blood sample such as a serum sample and other types of samples. In one embodiment, the sample is a blood derived sample, such as a whole blood, serum, or plasma sample. The sample may also be pretreated to increase the accessibility of target molecules, such as by lysis (mechanical, chemical, enzymatic cleavage, etc.), purification, centrifugation, separation and the like. The sample may also be labeled to facilitate detection of the presence of target molecules (fluorescence, radioactive, luminescence, chemical, enzymatic labeling, etc.).

In one embodiment, the biological sample according to the invention is a body fluid sample including blood. Liquid samples from various sources can be used for detection directly or after pretreatment by centrifugation, precipitation, filtration, etc.

In one embodiment, the biological sample according to the invention is a blood sample. In one embodiment, the blood sample is a whole blood sample, i.e. blood not subjected to a separation step, optionally diluted. In one embodiment, the blood sample according to the invention is a plasma or serum sample. The "plasma" as used in the invention refers to a liquid containing no cell component obtained by centrifugation after anti-coagulation treatment of ex vivo whole blood. The "serum" as used in the invention refers to a liquid which is released through blood clot condensation after ex vivo whole blood coagulates, and the serum does not contain fibrinogen. Since FN is involved in platelet adhesion, aggregation, and thrombus formation, the FN content in serum is generally considered to be lower than that in plasma. In one embodiment, the blood sample according to the invention is a plasma sample.

In a further embodiment, sodium citrate is used as an anticoagulant of the blood sample. In one embodiment, the blood sample is collected by using a siliconized or plastic syringe or a glass product coating with silicon, so as to avoid the activation of coagulation reaction. In one embodiment, the blood sample is a fresh or cryopreserved sample. In one embodiment, a freshly collected blood sample is directly diluted in 10× volume physiological saline or buffer, and a cell-free supernatant is taken as a sample. The treatment methods of various types of blood samples and/or the conversion relationship between the content of pED-B in a treated sample and the content of pED-B in whole blood is well-known to those skilled in the art.

The ED-B protein of the invention in a biological sample can be detected by directly or indirectly determining the level of the ED-B protein of the invention, such as pED-B, in a biological sample such as a blood sample (e.g. plasma). In one embodiment, the content of the ED-B protein may be determined by means of a specific binding agent, wherein the specific binding agent is an antibody.

Those skilled in the art are well aware that the ED-B protein fragment in blood can be detected by immunological techniques, including for example enzyme-linked immunosorbent assay (ELISA), AlphaLISA, immunoblotting, dot blotting, co-immunoprecipitation, and colloidal gold immunochromatograohic assay.

The full name of ELISA is enzyme-linked immunosorbent assay. There are many types of application methods based on ELISA, such as direct method, indirect method, double antibody sandwich method and the like, which are well-known to those skilled in the art. One of the applications is that, during an assay, a test sample (also called an antigen) reacts with a capture antibody on the surface of a solid support to form a capture antibody-antigen complex on the surface of the solid support. After washing and removing the free antigen, an enzyme labeled detection antibody is added, and the enzyme labeled detection antibody binds to the tested target on the surface of the solid support; at this time, a capture antibody-antigen-enzyme labeled detection antibody double antibody sandwich complex is formed, and the enzyme amount on the solid support is positively correlated with the amount of the target to be tested. After a substrate is added, a colored product is generated via enzyme catalysis, and the amount of the product is also correlated with the amount of a tested substance in the sample, so qualitative or quantitative analysis can be performed according to color shade. In another application, on the basis of forming an antibody-antigen complex on the surface of a solid support, an unlabeled detection antibody is added to form a capture antibody-antigen-detection antibody double antibody sandwich complex, then an enzyme labeled secondary antibody is added to form a capture antibody-antigen-detection antibody-enzyme labeled antibody complex, and subsequent detection is completed by means of enzymatic substrate. In another application, during assay, an antigen is immobilized on the surface of a solid support, and an enzyme labeled detection antibody is added to form an antigen-enzyme labeled detection antibody complex, and the detection is completed by means of enzymatic substrate. In another application, during assay, an antigen is immobilized on the surface of a solid support, a detection antibody is added to form an antigen-detection antibody complex, then an enzyme labeled secondary antibody is added to form an antigen-detection antibody-enzyme labeled antibody complex, and the detection is completed by means of enzymatic substrate. The capture antibody is used to capture the antigen to be tested, and the detection antibody is used to detect the total amount of the antigen captured by the capture antibody. The detection antibody may be directly labeled with an enzyme, or the secondary antibody against the detection antibody may be enzymatically labeled. An enzyme labeled is a product of an enzyme and an antibody or an antigen or a hapten under the action of a cross-linker and is a key reagent for the success of ELISA. It not only has an antibody and antigen-specific immune reaction, but also has an enzymatic reaction, and shows a biological amplification effect. However, different substrates are adopted for different enzymes. Enzymes commonly used in immunological techniques include horseradish peroxidase, alkaline phosphatase, glucose oxidase and β-D-galactosidase. Enzyme substrates commonly used in immunological techniques include o-phenylenediamine (OPD), tetramethylbenzidine (TMB), p-nitrophenyl phosphate (pNPP), and diaminobenzidine (DAB).

The antibodies used in ELISA can be either monoclonal or polyclonal, and can be obtained by animal immunization, or by genetic engineering techniques and computer-aided techniques. Preferably, monoclonal antibodies have higher specificity. The ELISA method can be used to detect liquid samples such as blood, sputum, cell culture broth, urine, tissue fluid and cerebrospinal fluid, as well as animal tissue, immobilized tissue and cells. For example, animal tissues or cells can be used for ELISA detection after homogenization through liquid nitrogen grinding and other methods.

In one embodiment, the concentration or content of the ED-B protein or ED-B protein fragment is detected through double antibody sandwich enzyme immunoassay. The ED-B protein or ED-B protein fragment in the biological sample, such as the blood sample, is captured by an ED-B specific capture antibody coated on an ELISA plate to highly enrich the ED-B protein or ED-B protein fragment, so as to facilitate the binding of an enzyme labeled ED-B specific detection antibody and improve detection sensitivity. FN is generally present in the form of a dimer in blood, and the "ED-B capture antibody coated on the ELISA plate" and the "enzyme labeled anti-ED-B specific detection antibody" used for blood FN detection by double antibody sandwich assay can be different antibodies, can also be antibodies which can recognize the same antigen epitope, or can also be the same antibody.

FN(B+) in the blood sample can be detected by double antibody sandwich ELISA. Two antibodies which recognize FN(B+) can be used, such as any two of non-competitive antibodies of CGS-1, CGS-2, L19, B5, BC-1 and C6 which recognize ED-B directly or indirectly. In addition, FN(B+) in the blood sample can be detected by sandwich ELISA, and one can use one ED-B specific antibody and one FN specific antibody.

The double antibody sandwich ELISA of the invention can be used to detect the ED-B protein or ED-B protein fragment or ED-B domain in blood, and can also be used to detect an ED-B protein fragment in tissue homogenate or cell lysis solution. Since FN is a long fibrillar protein, has multiple proteolytic sites and is easy to break into small fragments, ED-B or ED-B-containing proteins can be detected by two antibodies directly acting on ED-B, and ED-B detection sensitivity and accuracy can be improved.

In one embodiment, the ED-B protein or ED-B protein fragment comprises the amino acid sequence as set forth in SEQ ID No. 1 or a sequence having at least 80%, 85%, 90%, 95%, 96%, 97%, 98%, 99% or more sequence identity with SEQ ID No. 1. The ED-B amino acid sequence is encoded by a DNA sequence as set forth in SEQ ID NO. 2. The ED-B amino acid sequence can be encoded by a DNA sequence as set forth in SEQ ID NO. 3. In one embodiment, the ED-B protein is FN containing the ED-B domain.

In another embodiment, the concentration or content of the ED-B protein or ED-B protein fragment can also be detected through AlphaLISA. AlphaLISA is based on the principle of the interaction of biomolecules. For example, antigen can interact with antibody, and antibody and microbead can form a microbead complex through the interaction between molecules, wherein one of the antibodies and the microbead containing a photosensitizer are bound to form a donor microbead, the other antibody is bound to the microbead containing a dimethyl thiophene derivative which can react with monomeric oxygen to form an acceptor microbead, and a donor microbead-antigen-acceptor microbead complex can be formed in the presence of an antigen. The donor microbead is excited by laser to release singlet oxygen molecules, initiating an energy transfer cascade reaction which causes the acceptor microbead to emit light waves, and the light waves have a strong anti-quenching capability and can be received by a detector. If there is no specific interaction between biomolecules, the donor microbead-antigen-acceptor microbead complex cannot form, monomeric oxygen cannot diffuse into the acceptor microbead, and no optical signal can be generated. Like double antibody sandwich ELISA, AlphaLISA also needs the formation of a double antibody sandwich complex, but AlphaLISA has higher sensitivity, uniformity and wider dynamic detection range than ELISA, low demand for sample and easy operation, and does not require washing. The ED-B protein of the invention can be detected more rapidly, efficiently and sensitively by AlphaLISA.

In another embodiment, the concentration or content of the ED-B protein or ED-B protein fragment can also be detected through Pull-Down method. The basic principle of the Pull-down technique is to immobilize a ligand with an affinity for a target protein on a substrate (such as Sepharose), when a solution containing the target protein passes through the substrate, the target protein interacts with the immobilized affinity ligand and is adsorbed by the substrate, non-target proteins flow out with the eluent, and the target protein can be recovered by changing elution conditions. Specifically, by binding an ED-B specific antibody to a synthetic resin and making a processed sample, such as a blood sample, flow through the resin, the antibody on the resin captures the ED-B protein in the sample, non-ED-B proteins flow out, and non-specific proteins on the resin can be further removed through washing or other methods. Quantitative determination of ED-B, such as pED-B can be performed through SDS-PAGE, or Western blot, or ELISA, or ultraviolet detection, etc. on the ED-B protein adsorbed on the resin.

In another embodiment, the detection of the ED-B protein comprises non-specifically concentrating the proteins containing ED-B domain in the liquid, i.e. enriching the protein and then detecting.

In another embodiment, the concentration or content of the ED-B protein or ED-B protein fragment is detected through dot blot of the protein. Dot blot is to apply a sample to be tested to a membrane, fix the protein on the membrane or other solid phase materials by air-drying, baking, ultraviolet irradiation, etc., and then detect an antibody or color development of a corresponding enzyme and substrate so as to analyze qualitatively or quantitatively. The method can enrich large amount and high-concentrated proteins on the membrane, which can make the target protein highly enriched and facilitate detection. The method is less time-consuming than Western blot, and multiple samples can be detected simultaneously on one membrane, which can be used as a gene chip. Although Western blot can also be used as one of the detection methods for pED-B, SDS-PAGE and other electrophoresis methods can separate proteins of different molecular weights or different charge distributions, and the amount of sample to be loaded during detection is limited, as a result, the detection sensitivity of pED-B will be reduced during pED-B protein detection, especially, the molecular weights of the pED-B protein may vary greatly, and different band distributions may occur during Western blot. By this detection method, the sample can be concentrated by applying the sample on the membrane multiple times and drying the sample, so as to improve the sensitivity of detection, and the applied sample can be further enriched and concentrated by multiple times of sample application and air-drying so as to detect low-concentration proteins. Dot blot and Western blot as described herein are well-known to those skilled in the art.

In another embodiment, the concentration or content of the ED-B protein or ED-B protein fragment is detected through blood smear. The procedure is similar to that of dot blot. The preparation of blood smear is well known to those skilled in the art.

In another embodiment, the concentration or content of the ED-B protein or ED-B protein fragment is detected through colloidal gold immunochromatographic assay. Colloidal gold immunochromatograohic assay is to fix a specific antibody (or antigen) on a membrane in the form of strip, a colloidal gold labeling reagent (antibody) is adsorbed on a binding pad, when a sample to be tested is added to a sample pad at one end of test strip, the sample moves forward under the capillary action to dissolve and react with the colloidal gold labeling reagent on the binding pad, when moving to the fixed antibody (or antigen) region, a conjugate of the sample to be tested and the gold labeling reagent specifically binds to the fixed antibody (antigen) and is trapped and gathered on detection band, and the color development can be observed visually. Diagnostic strip has been designed based on this method and very convenient to use.

It is well known to those skilled in the art that the anti-ED-B antibody used, such as the anti-pED-B antibody, can be coupled to horseradish peroxidase, and can also be coupled to alkaline phosphatase; and the indirect detection of ED-B, such as pED-B, can also be achieved by means of a secondary antibody coupled to the peroxidase or alkaline phosphatase described above. Reaction substrates include DAB, TMB, or fluorescent substrates.

In another aspect, the invention also relates to a method of detecting pED-B. Since pED-B is first detected in blood, few methods have been validated for pED-B detection, and Western blot as reported failed to detect pED-B (Viti, Tarli et al. 1999. Cancer Res. 59: 347-352). It is presumed that since the content of pED-B is low and FN easily aggregates and precipitates in blood, its detection is difficult. The pED-B detection method of the invention employs the pED-B protein enrichment technique and improves pED-B detection sensitivity. One method of pED-B enrichment is to use a specific method to capture and enrich pED-B on a solid support, and non-pED-B proteins can be removed by washing, etc., for example, pED-B can be captured by means of a pED-B specific antibody; another method is to use a non-specific method to fix pED-B and non-pED-B protein mixture on the surface of a solid support, but detect the content of pED-B by means of a specific antibody; in addition, pED-B can also be directly detected by mass spectrometry.

In one embodiment, the invention provides a method for detecting the level of ED-B protein, such as pED-B protein, in a sample, such as a blood derived sample, comprising:
- contacting a first ED-B protein binding agent, such as an antibody, with the sample to be detected, optionally, the first ED-B protein binding agent is immobilized on a solid support, such as an ELISA plate;
- adding a second ED-B protein binding agent, such as an antibody, optionally labeled, for example conjugated with an enzyme (e.g. horseradish peroxidase, alkaline phosphatase, glucose oxidase, β-D-galactosidase), optionally, the second ED-B protein binding agent and the first ED-B protein binding agent bind to different epitopes on the ED-B protein;
- optionally, adding an antibody against the second ED-B protein binding agent, optionally labeled, for example conjugated with an enzyme (e.g. horseradish peroxidase, alkaline phosphatase, glucose oxidase, β-D-galactosidase); and
- detecting the formed complex.

The detection may be a chromogenic reaction known in the art, for example using the substrate o-phenylenediamine (OPD), tetramethylbenzidine (TMB), p-nitrophenyl phosphate (pNPP), diaminobenzidine (DAB), etc. In one embodiment, the first and second ED-B protein binding agents are binding agents specific for ED-B protein, for example, independently selected from B5 and L19.

In another aspect, the invention provides use of a binding agent which specifically detects the ED-B protein for the detection of ED-B protein in a blood sample, of a mammal such as human being.

The binding agent which specifically detects the ED-B protein may be used in preparation of a composition or kit for detecting the level of the ED-B protein in a blood sample of a mammal (such as human being) to diagnose the presence of or the risk of tissue hyperplasia and solid tumors in the mammal, perform prognosis of solid tumors, determine the occurrence or progression of solid tumors, perform screening of solid tumors, or monitor the efficacy of treatment of solid tumors. In one embodiment, the mammal is human being.

In another aspect, the invention provides a kit for detecting ED-B protein in a blood sample, of a mammal such as human being, the kit comprising the binding agent specifically detecting the ED-B protein in the blood sample of a mammal. In one embodiment, the mammal is human being.

The substance specifically detecting the ED-B protein according to the invention is a binding agent specific for the ED-B protein, such as pED-B, which aids in, by detecting the level of the ED-B protein such as pED-B in a biological sample, such as a body fluid sample, preferably blood, plasma or serum, determining the occurrence or progression of tumors, performing tumor screening on high-risk groups, or the prognosis of tumor patients, or evaluating whether the treatment on tumor patients is effective, or personalized medication.

Aided diagnosis refers to using serology, iconography and pathology to assist physicians in judging the type and severity of a disease and improving the accuracy of diagnosis.

Prognosis refers to predicting the possible course and outcome of a disease. It includes determining the specific consequences of the disease and predicting the likelihood of a certain outcome over time.

Personalized medication refers to making a safe, rational, effective and economical drug treatment regime based on the characteristics of individual patients, such as hereditary factors, gender, age, weight, physiological and pathological characteristics, and other drugs which are being taken.

The "binding agent specific for ED-B protein" in the invention refers to a molecule capable of recognizing and binding to the ED-B protein or ED-B protein fragment described in the invention, the molecule having specificity and high affinity to the ED-B protein or ED-B protein fragment described in the invention, and also includes molecules which recognize the ED-B protein or fragment on a cell surface or extracellular matrix with high affinity and specificity. The specific binding agent may be a protein or a nucleic acid.

In one embodiment, the binding agent specific for the ED-B protein of the invention is an antibody specific for the ED-B protein such as pED-B. In one embodiment, the antibody is polyclonal antibody or monoclonal antibody or an antigen-binding fragment of an antibody, for example, a polypeptide containing antibody variable regions or variable region sequences, scFv, Fab, Fab' and F(ab')₂. In one embodiment, the antibody of the invention may be an antibody obtained by immunizing an animal, such as a mouse, rat or rabbit. In one embodiment, the antibody of the invention can be designed by computer simulation, or with artificial assistance based on an antibody obtained by immunization, or obtained by means of gene synthesis.

Because the ED-B domain is highly conserved in different species, the ED-B amino acid sequences of human, mouse, rat, dog and rabbit are identical and highly homologous to chicken ED-B. The ED-B of the invention is highly homologous in mammals and therefore has similar biological characteristics in different species. Specific antibodies directly recognizing an ED-B surface antigen are generally not species-specific, such as CGS-1, CGS-2 (PCT/GB97/01412; Nissim, Hoogenboom et al. (1994). EMBO J. 13: 692-698), L19 (Pini, Viti et al. (1998). J Biol Chem. 273: 21769-21776), B5 (CN201480001324.5), etc. Kits developed based on the above antibodies can also be used for samples of different species origins. Specific antibodies which indirectly recognize ED-B may be species-specific, such as BC-1 (Carnemolla, Balza et al. (1989). J Cell Biol. 108: 1139-1148), C6 (Ventura, Sassi et al. (2010). PLoS One. 5: e9145.) and others which can recognize epitopes on domains adjacent to the ED-B in human.

The kit according to the invention can be used for detecting the content of the protein fragment of the invention in a biological sample such as a body fluid sample, preferably a blood derived sample. In one embodiment, the kit according to the invention comprises at least two antibodies to the ED-B protein. The kit of the invention comprises an ED-B specific antibody, such as L19 and/or B5. In one embodiment, the L19 and B5 antibodies are capture and enzyme labeled antibodies respectively, for detection of the ED-B protein or ED-B protein fragment. The binding of one of the two antibodies to ED-B does not affect the binding of the other to ED-B, suggesting that the antigen epitopes of the L19 and B5 antibodies binding to ED-B are not the same. Since FN(B+) also has the biological characteristic of forming dimers, even antibodies which recognize the same epitope can be used for the development of the kit.

The composition or kit of the invention can be used for tumor screening in high risk groups by detecting the level of the ED-B protein of the invention, such as pED-B, in a biological sample, such as a body fluid sample, preferably a blood derived sample, assessing the probability that a sample provider has a tumor by measuring the content of the ED-B protein, such as pED-B, in a sample of a suspected tumor patient, and comparing with a healthy control. The more increase in the level of the ED-B protein such as pED-B than the normal level, the higher the probability that the sample provider has a tumor. In another embodiment, the method can also be used to determine if a subject has a tumor.

The composition or kit of the invention can be used for performing the prognosis of a tumor patient by detecting the level of the ED-B protein, such as pED-B, in a biological sample, such as a body fluid sample, preferably a blood derived sample, providing the prognosis of a sample provider by measuring the content of the ED-B protein, such as pED-B, in a sample of a tumor patient, and comparing with a healthy control or the content of the ED-B protein, such as pED-B, in a previous corresponding sample of the patient. A continuous high level of the ED-B protein, such as pED-B may be associated with an unfavorable prognosis.

The composition or kit of the invention can be used for assessing the treatment effect on a tumor patient by detecting the level of the ED-B protein of the invention, such as pED-B, in a biological sample, such as a body fluid sample, preferably a blood derived sample, assessing the treatment effect on a sample provider by measuring the content of the ED-B protein, such as pED-B, in a sample of a tumor patient, and comparing with a healthy control or the content of the ED-B protein, such as pED-B, in a previous corresponding sample of the patient. The level of the ED-B protein, such as pED-B, is used to aid in determining whether to maintain the current treatment or alter the treatment strategy.

The composition or kit of the invention can be used for performing personalized medication by detecting the level of the ED-B protein of the invention, such as pED-B, in a biological sample, such as a body fluid sample, preferably a blood derived sample, assessing the treatment effect on a sample provider by measuring the content of the ED-B protein, such as pED-B, in a sample of a tumor patient, and comparing with a healthy control or the content of the ED-B protein, such as pED-B, in a previous corresponding sample of the patient. The level of the ED-B protein, such as pED-B, is used to aid in determining whether to maintain the current treatment or alter the treatment strategy, so as to perform personalized medication.

In one embodiment of the invention, the tumor is a solid tumor. The solid tumor can be a benign tumor, a malignant tumor and a borderline tumor. The malignant tumor can be a cancer or sarcoma, for example, various squamous cell carcinomas, adenocarcinomas and sarcomas. In one embodiment, the tumor is selected from the group consisting of esophageal cancer, nasopharynx cancer, cardia cancer, laryngeal cancer, head and neck cancer, gastric cancer, liver cancer, biliary tract cancer, gallbladder cancer, colon cancer, duodenal cancer, lung cancer, bladder cancer, cervical cancer, ovarian cancer, endometrial cancer, breast cancer, melanoma, pancreatic cancer, kidney cancer and prostate cancer. In one embodiment, the tumor is an upper gastrointestinal cancer, for example, selected from esophageal cancer, cardia cancer, laryngeal cancer and gastric cancer. In one embodiment, the tumor is nasopharyngeal cancer, esophageal cancer, gastric cancer, lung cancer or pancreatic cancer.

In one embodiment, the kit of the invention comprises monoclonal antibody B5 or L19 which specifically recognize the ED-B domain of FN with high affinity and sensitivity. The invention also develops an ELISA detection kit based on the detection method for pED-B in blood. ELISA is a commonly used protein immunoassay method, but because of few antibody types satisfying the requirements of ELISA kit development, low commercialization and difficulty in obtaining, it has not been possible to establish an effective method for detecting ED-B in blood, and there are few reports concerning pED-B. In one embodiment, the kit of the invention comprises both B5 and L19 antibodies as candidate antibodies for double antibody sandwich ELISA.

In one embodiment, the kit according to the invention further comprises an anticoagulant capable of preventing blood coagulation, such as natural anticoagulant heparin or hirudin, and divalent calcium ion chelating agent sodium citrate or ethylenediaminetetraacetic acid (EDTA). An anticoagulant-treated blood product or plasma can be used in the ED-B detection method according to the invention. Preferably, the invention employs sodium citrate as anticoagulant when obtaining plasma, so as to reduce the precipitation loss of FN.

The invention discovers that the ED-B protein is relatively stable in healthy humans and is substantially increased in the blood of tumor patients and tissue hyperplasia patients, suggesting that pED-B is closely related to the occurrence and development of tumors. Therefore, the ED-B protein of the invention can serve as a tissue hyperplasia and tumor marker. As a tumor blood marker, pED-B can be widely used in medical diagnosis, treatment and scientific research. The detection of the level of the marker of the invention in blood is helpful to determine whether there is a tumor in the body for use in tumor screening in high-risk groups, or helpful to assess the progress of a tumor, or helpful to determine the prognosis of tumor treatment, or helpful for personalized medication and precise treatment, or for use in biological study of tumors.

### Examples

The invention is further illustrated by the following examples, but any examples or combinations thereof should not be construed as limiting the scope or implement of the invention. The scope of the invention is defined by the appended claims, and the scope defined in the claims can be clearly understood by those skilled in the art in combination with the specification and common knowledge in the art. Without departing from the scope of the invention, those skilled in the art can make any modifications or changes to the technical solutions of the invention, and such modifications and changes are also included within the scope of the invention.

### Example 1: Expression of the serum marker standard and antibodies

### 1. Vector construction

A) Serum marker standard vector construction: Synthesizing the DNA sequence of SEQ ID NO: 3, wherein the DNA sequence encodes three domains FN(7B8) of FN, and the encoded amino acid sequence may be found in Schiefner, Gebauer et al. (2012). J Biol Chem. 287: 17578-17588. In addition to encoding FN(7B8), the DNA sequence also encodes a signal peptide at the N-terminus of FN(7B8) and six His tags at the C-terminus, wherein the signal peptide facilitates the protein secretory expression of FN in mammalian cells, and the His tags are good for protein purification. The N-terminus and C-terminus of the sequence are cloned into pCI-neo vector (Promega Corporation) via Nhe I and Not I restriction endonuclease recognition sites, respectively, to form pCI-neo-7B8 vector.
B) Protein expression of B5 and L19 antibodies. Synthesizing the DNA sequences of B5 and L19 single-chain antibody fragment (scFv) structures (see Chinese Patent Application No. CN201480001324.5; Pini, Viti et al. (1998). J Biol Chem. 273: 21769 -21776 and Borsi, Balza et al. (2002). Int J Cancer. 102: 75-85) by gene synthesis. Adding a DNA sequence encoding a signal peptide to the 5' end and a DNA sequence encoding an IgG1 Fc tag to the 3' end of the DNA sequence of each antibody, wherein the signal peptide is used for protein secretory expression in mammalian cells, and IgG1 Fc is good for stability and protein purification of the antibodies (Cao, Cao et al. (2009). Appl Biochem Biotechnol. 157: 562-574). The N-terminus and C-terminus of the sequence are subjected to cleavage of Nhe I and Not I restriction endonucleases and clone into pCI-neo vector to form pCI-neo-B5-Fc vector and pCl-neo-L19-Fc vector. The antibodies described in this example are all in the form of a single-chain antibody. The antibody Fc fragment contains a hinge region and the obtained antibody has a molecular weight of approximately 52 kD. The antibodies described in the examples of the invention generally contain an Fc protein structure. The vector construction and expression of antibody proteins are commonly used in the art (Borsi, Balza et al. (2002). Int J Cancer. 102: 75-85).
C) Methods for the expression and purification of L19-IL2 refer to Carnemolla, Borsi et al. (2002). Blood. 99: 1659-1665.

### 2. Protein expression

After extracting a large amount of obtained plasmid containing the antibody fusion protein, transfecting the plasmid into CHO-K1 cells using the Invitrogen liposome reagent Lipofectamine^{®} 2000, and after selective culturing continuously for 4 weeks in a selective medium (Davies and Jimenez (1980).Am J Trop Med Hyg. 29: 1089-1092.) containing G418 by means of the neo gene of pCI-neo plasmid vector, performing cloning culture by limiting dilution method, and continuing to perform cloning culture of obtained monoclonal cells to obtain stable strains.

The obtained cell strains are expressed in suspension with Hyclone CD4CHO. The FN(7B8) protein is subjected to affinity purification with a nickel column, and the Fc-tagged antibody protein is purified with Protein A. The purified protein is subjected to SDS-PAGE to obtain the purity of the sample, the concentration of the sample is detected with a UV spectrophotometer, and the binding ability of FN(7B8) to the antibodies is evaluated by ELISA.

### Example 2: Detection of FN(B+) in plasma using L19-IL2 antibody fusion protein and B5-Fc antibody (sandwich ELISA)

FN(B+) in the blood sample can be detected by sandwich ELISA. Two antibodies which recognize FN(B+) can be used, such as any two non-competitive antibodies of CGS-1, CGS-2, L19 and B5 which recognize ED-B directly or indirectly. In addition, FN(B+) in blood sample can be detected by sandwich ELISA, using one FN specific antibody and one ED-B specific antibody.

The procedure for the detection of an FN(7B8) standard and a blood sample using L19-IL2 and B5-Fc antibodies is briefly described as follows:
1. Coating: an ELISA plate was coated with L19-IL2, 5 parallel samples. The capture antibody L19-IL2 was diluted with carbonate buffer (Na₂CO₃ 1.59 g/L, NaHCO₃ 2.93 g/L) at pH=9.6 to a concentration of 2 ng/µl, and the ELISA plate was coated at 100µl/well. The ELISA plate was placed in a biochemical incubator at 37°C for 2 h. The coated ELISA plate was washed 3 times with PBST (NaCl 8g/L, KCI 0.2g/L, Na₂HPO₄ 1.42g/L, KH₂PO₄ 0.27g/L, pH=7.4, 0.5‰ Tween-20) at 300µl/well, 5 min for each time. The ELISA plate was blocked with PBS containing 4% BSA (NaCl 8g/L, KCI 0.2g/L, Na₂HPO₄ 1.42g/L, KH₂PO₄ 0.27g/L, pH=7.4) at 300µl/well, for 1h at 37°C. The ELISA plate was washed 3 times with PBST at 300µl/well, 3 min for each time.
2. Adding the standard: 100µl of FN(7B8) standard were added to the wells of the ELISA plate at an amount of 0.25, 0.5, 1, 2, 4, 8, 16, 32ng of protein/well respectively.
   Adding the sample to be tested: adding the serum sample diluted with PBS by 50 folds to the sample wells of the ELISA plate at 100µl/well. BSA was used as a negative control. The ELISA plate was incubated at 37°C for 1 h.
3. Adding the primary antibody: the ELISA plate was washed 3 times with PBST at 300µl/well, 3 min for each time. The primary antibody was B5-Fc. Each antibody was diluted to 2ng/µl with PBST, and added to the sample wells of the ELISA plate at 100µl/well, followed by incubation at 37°C for 1 h.
4. Adding the secondary antibody: the ELISA plate was washed 3 times with PBST at 300µl/well, 3 min for each time. The secondary antibody was HRP-conjugated mouse anti-human IgG antibody (Boster BA1070), and was diluted by 5000 folds with PBST, added to the sample wells of the ELISA plate at 100µl/well and incubated at 37°C for 1 h.
5. Adding the substrate for development: the ELISA plate was washed 5 times with PBST at 300µl/well, 3 min for each time. TMB developing solution was added to the sample wells at 100µl/well, and incubated at room temperature for 5 min.
6. Stopping the reaction: the reaction was stopped with 2mol/L H₂SO₄, the stop solution was added to the sample wells at 100µl/well to stop the reaction, and the color of the sample wells changed from blue to yellow immediately.
7. Detecting the absorbance value: the detection was carried out at OD450nm and OD630nm wavelength detection with microplate reader. The sample detection absorbance value was obtained after the background value of the blank control well was subtracted.

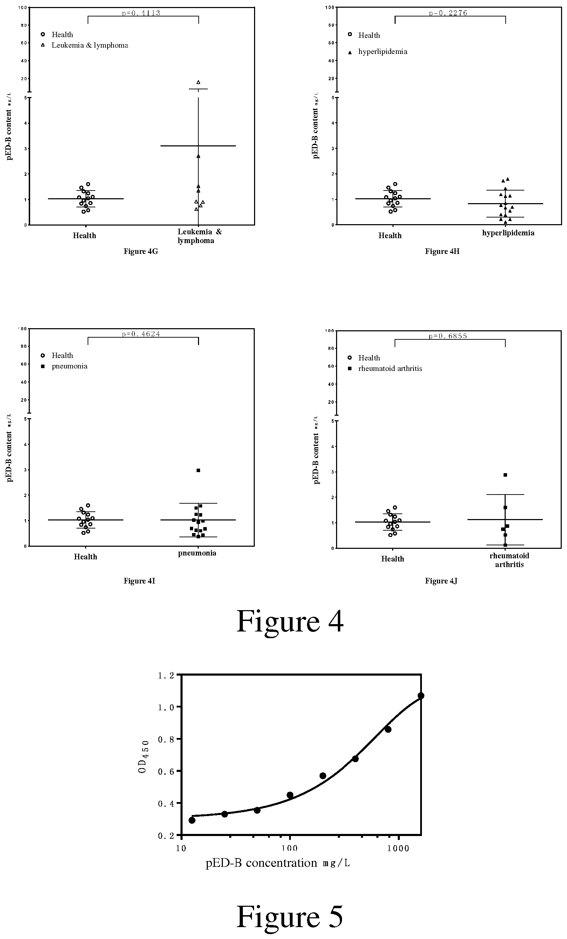

From the absorbance value obtained by indirect detection of the enzyme-labeled mouse anti-human antibody, the value of the combination of B5 and L19 was significantly higher than that of the negative control, i.e. the sensitivity of the combination of B5 and L19 antibodies satisfies the requirement of pED-B detection. The result was shown in FIG. 5.

### Example 3: Mouse tumor model establishment and FN(B+) detection

Twelve healthy balb/c nu nude mice were reared to 20g/mouse in an SPF barrier system, wherein there were six mice in the experimental group, human pharyngeal squamous cell line FaDu (Rangan (1972).Cancer. 29: 117-121) was implanted subcutaneously in the back of each mouse (about 2 million cells), and cultured continuously. When the mouse tumor grew to an average diameter of 2 cm (about 4 weeks), the whole blood of the mouse was collected, using sodium citrate as anticoagulant, centrifuged at 4°C and 3000g for 2 min, and the plasma supernatant was removed and the precipitate was discarded, repeating once, and the content of ED-B in plasma was measured with the ELISA method.

The plasma samples of six healthy balb/c nu mice were used as control samples to detect the content of FN(B+) in the blood of mice.

The method for detecting the content of FN(B+) in the blood of mice comprised, in brief, coating a COSTER 96-well ELISA plate with L19-Fc antibody (2ng/µl, 100µl/well) at 37°C for 2 h, blocking overnight with a PBS solution containing BSA at 4°C, washing 3 times with PBS, adding 100µl of mouse plasma diluted with PBS by 10 times and incubating at room temperature for 2 h, adding an HRP-conjugated B5-Fc antibody (2ng/µl, 100µl/well) after sufficient washing, and developing using the TMB method.

The results showed that the content of ED-B in the blood of healthy mice was 0.35±0.15 mg/L, suggesting that there is ED-B in the blood of healthy mice, or FN(B+) in the blood samples of healthy mice.

The content of ED-B in the blood of tumor-bearing mice was 0.66±0.25 mg/L. Compared with healthy mice, the content of ED-B in the plasma of tumor-bearing mice was increased.

### Example 4: Human plasma sample collection, and determination of pED-B and tumor malignancy by pull-down assay

The blood from healthy people and patients with tumors was collected, treated with the anticoagulant sodium citrate, centrifuged at 4°C and 3000g for 2 min, and the plasma was removed and the precipitate was discarded, repeating once.

50µl of Protein A (Genscript Co.) packing material was pretreated with Binding wash buffer, then transferred to a 1.5ml centrifuge tube, and 0.2mg of B5-Fc antibody was added, and inversion mixing at 4°C for 1h, the Protein A and B5-Fc antibody dimer complex was formed. The supernatant was removed after centrifugation, Protein A was washed 3 times with Binding wash buffer, discarding the supernatant washing solution, and the Protein A and B5-Fc antibody dimer complex was equally divided into three parts, and added to three samples respectively, wherein A) 100µl healthy plasma, B) 100µl esophageal cancer patient plasma, and C) BSA solution, negative control. Inversion mixing at 4°C for 1 h and washing 3 times with PBS. The complex precipitate was taken for sample processing for SDS-PAGE detection.

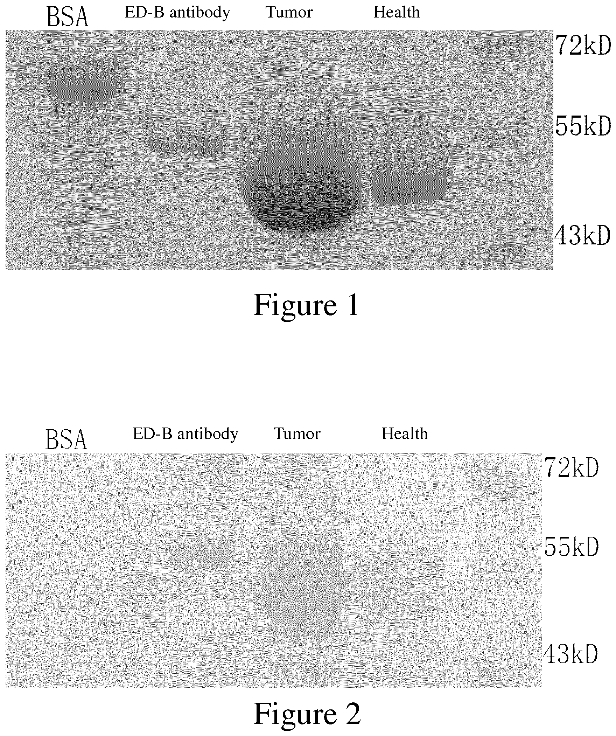

The concentration of separation gel of the SDS-PAGE was 8%, the concentration of concentration gel was 5%, the sample loading volume was 20µL, the current was 30mA, and the Coomassie brilliant blue was used for staining. From the results of SDS-PAGE detection, B5-Fc captured protein was detected in the plasma of both healthy people and the selected tumor patients, and the concentration of the protein captured from the plasma samples of tumor patients was significantly higher than that of healthy people. See FIG. 1.

In addition, Western blot analysis was performed based on the above SDA-PAGE experiment, and BSA was used as negative control. Anti-ED-B antibody L19-Fc was used as the primary antibody, horseradish peroxidase-labeled goat anti-human IgG antibody was used as the secondary antibody, and the development was achieved with the substrate DAB. Western blot results indicated that the protein captured by the Protein A and B5-Fc antibody dimer complex was the ED-B domain containing protein. The Protein A and B5-Fc antibody dimer complex cannot bind to the proteins in the BSA solution. See FIG. 2.

### Example 5: Relationship of determination of the content of ED-B in blood and tumorigenesis

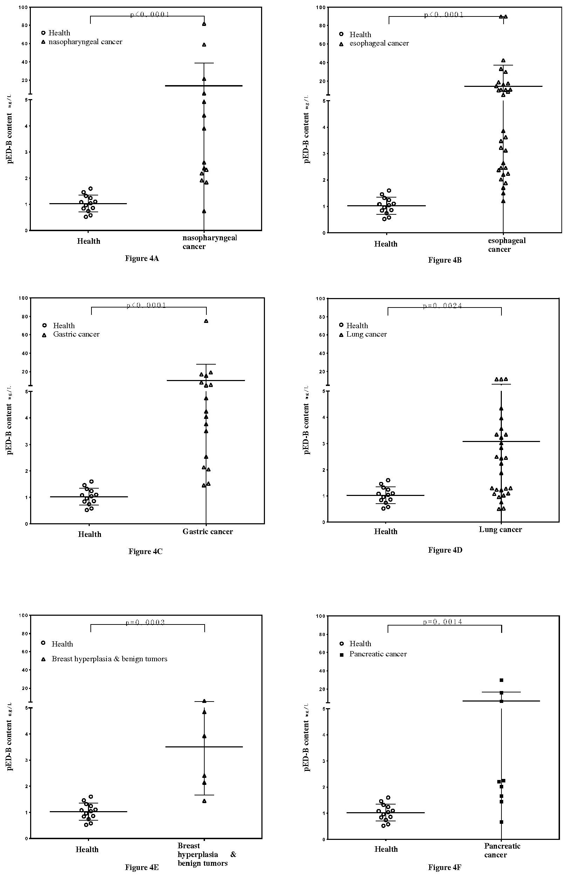

The ELISA method described in Embodiment 2 was used to detect the content of ED-B in the plasma of healthy people and newly diagnosed tumor patients (including the patients with nasopharyngeal carcinoma, esophageal cancer, gastric cancer, lung cancer, pancreatic cancer, leukemia and lymphoma, later confirmed by pathological or radiological diagnosis), benign tissue hyperplasia (including breast hyperplasia, benign tumors) and some chronic diseases (including hyperlipidemia, rheumatoid arthritis, pneumonia) (see FIG. 4A-J).

The results showed that the content of ED-B in healthy human blood was relatively stable, the level of ED-B in the blood of patients with solid tumors was significantly increased, and the effects of different tumor types on the level of ED-B elevation were different. For example, the level of pED-B in the blood of patients with esophageal cancer, gastric cancer, nasopharyngeal cancer and lung cancer was significantly increased, and was statistically significant compared with healthy individuals. The difference of the level of pED-B in the blood of patients with leukemia, lymphoma, hyperlipidemia, pneumonia and rheumatoid arthritis from that of healthy individuals was not statistically significant. The level of pED-B in the blood of patients with benign tumors and breast hyperplasia was also increased, and was statistically significant compared with healthy individuals. The statistical method was Mann-Whitney test.

### Example 6: Preparation of ELISA kit for detecting ED-B

An ED-B ELISA kit consists of an ELISA plate coated with L19-Fc antibody; horseradish peroxidase (HRP)-conjugated B5-Fc antibody; ED-B standard; sample diluent; washing solution; developing agent; and stop solution.

The above solutions were prepared as follows:
1. ELISA plate coated with L19-Fc antibody
   1) Coating: the capture antibody was L19-Fc, and was diluted with carbonate buffer of pH=9.6 to a concentration of 2ng/µl, and the ELISA plate was coated at 100µl/well (200ng/well). The ELISA plate was incubated in a biochemical incubator at 37°C for 2h, and then in a 4°C refrigerator for 8-10 h.
   2) Washing: the coated ELISA plate was washed 5 times with PBST at 300µl/well, 3 min for each time.
   3) Blocking: the ELISA plate was blocked with 4% BSA, 300µl/well, for 1 h at 37°C.
   4) Washing: the ELISA plate was washed 5 times with PBST at 300µl/well, 3 min for each time.
   5) Air-drying: finally, the ELISA plate was placed in a cool place for air-drying, and then stored in a 4°C refrigerator for later use.
2. Horseradish peroxidase (HRP)-conjugated B5-Fc antibody: sugar molecules on the surface of HRP were oxidized into aldehyde group using NaIO₄, then HRP binds to an amino group on the antibody. The yield of the enzyme-labeled antibody obtained by this method was high, and there was no significant loss of activity of the enzyme and the antibody (Tsang, Greene et al. (1995). J Immunoassay. 16: 395-418).
3. ED-B standard: FN(7B8) standard
4. Sample diluent

PBS: NaCl 8g, KCI 0.2g, Na₂HPO₄ 1.42g, KH₂PO₄ 0.27g, making the volume into 1L with distilled water, adjusting pH to 7.4, and filtering.

### 5. Washing solution

PBST: 1L, i.e. Tween-20 was added to the PBS in a volume ratio of 0.5%o, and mixing.

### 6. Preparation of developing agents

Substrate developing solutions include solution A and solution B, which are mixed in equal volumes before use, and developing in the dark.

The preparation process of the solution A is: dissolving 1g of urea peroxide, 35.8g of disodium hydrogen phosphate and 10.2g of citric acid into ddH₂O in the dark, then adding 100µl of Tween-20, to a final volume of 1L, and keeping the solution at 4°C in the dark;

The preparation process of the solution B is: dissolving 0.7g of TMB and 10.3g of citric acid in 40ml of DMSO in the dark, then adding ddH₂O to a final volume of 1L, and keeping the solution at 4°C in the dark.

7. Preparation of stop solution: 98% concentrated sulfuric acid and ultrapure water were mixed in a volume ratio of 1:8 to obtain a 2mol/L sulfuric acid solution.

pH=9.6 carbonate coating solution: 0.05M carbonate buffer, and the formula is Na₂CO₃ 1.59 g and NaHCO₃ 2.93 g dissolved in 1L of deionized water.

### Example 7: detecting the content of ED-B in various samples using the ELISA kit of the invention

### 1. Test samples and treatment

1) Serum: 20-week-old Bal B/C mouse fresh blood was collected, placed in a 1.5mL centrifuge tube, and tilted or laid flat in a biochemical incubator at 37°C for 1 h. After the serum was separated, centrifugation was performed (3000 g, 5 min) twice, and the supernatant was pipetted (be careful, not to aspirate red blood cells). The supernatant was used for ELISA detection. The sample to be tested was placed on ice or in a 4°C refrigerator for a short period of time, and for long-term storage, frozen at -20°C or below.
2) Plasma: during collection of the fresh blood from 20-week-old Bal B/C mice, an appropriate amount of anticoagulant sodium citrate was added, a blood collection tube was vertically placed for 1 h so that the blood was naturally precipitated, and then centrifuged in a centrifuge tube twice (3000 g, 5 min), and the plasma was separated for detection. The sample to be tested was placed on ice or in a 4°C refrigerator for a short period of time, and for long-term storage, frozen at -20°C or below.
3) Blood sample: the fresh blood of 20-week-old Bal B/C mice was collected, diluted by 100 folds with PBS, and centrifuged (3000 g, 5 min) twice, and the supernatant was taken for ELISA detection. The sample to be tested was placed on ice or in a 4°C refrigerator for a short period of time, and for long-term storage, frozen at -20°C or below.
4) Cell sample treatment: cultured adherent FaDu cells were scraped off and then transferred to a centrifuge tube, and suspended cells were directly transferred to a centrifuge tube. The cells were centrifuged at 1000 g for 5 min, and washed three times with PBS, then PBS was discarded. Liquid nitrogen was added and ground with a grinding rod, then an appropriate amount of PBS was added, centrifuged (3000 g, 5 min) twice, and the supernatant was taken for ELISA detection. The sample to be tested was placed on ice or in a 4°C refrigerator for a short period of time, and for long-term storage, frozen at -20°C or below. The total protein concentration was determined by the BCA method.
5) Tissue sample treatment: the fresh sample collected from Bal b/c nude mouse transplanted with human pharyngeal squamous cell (FaDu) was washed to blood stains, a protease inhibitor was added as required, and animal tissue was cut into small pieces and added to liquid nitrogen, and ground in a mortar into fine powder. An appropriate amount of PBS was added and centrifuged (3000 g, 5 min) 2 times, and the supernatant was taken for ELISA detection. The sample to be tested was placed on ice or in a 4°C refrigerator for a short period of time, and for long-term storage, frozen at -20°C or -80°C. The total protein concentration was determined by the BCA method.

### 2. Testing steps:

1) Adding the sample to be tested: adding the diluted sample to sample wells of the ELISA plate at 100µl/well, and the standard protein EDB (a series of 2-fold dilutions, 32-0.25 ng/well) to establish a standard curve. The negative and blank controls were made at the time. The ELISA plate was incubated at 37°C for 1 h.
2) Washing: the ELISA plate was washed 5 times with PBST at 300µl/well, 3 min for each time.
3) Adding an enzyme labelled antibody: the antibody was diluted to 2ng/µl with PBST, and added to the sample wells of the ELISA plate at 100µl/well, followed by incubation at 37°C for 1 h.
4) Washing: the ELISA plate was washed 5 times with PBST at 300µl/well, 3 min for each time.
5) Adding a substrate for development: TMB developing solution was added to the sample wells at 100µl/well and incubated at room temperature for 5-6 min.
6) Stopping the reaction: the reaction was stopped with 2M H₂SO₄, and the stop solution was added to the sample wells at 100µl/well to stop the reaction, and the color of the sample wells changed from blue to yellow immediately.
7) Detecting the absorbance value: the detection at both 450nm and 630nm wavelength detection was done with microplate reader. The sample detection absorbance value was obtained after the background value of blank control well was subtracted.
8) Data processing. Expressed as mean+variance.

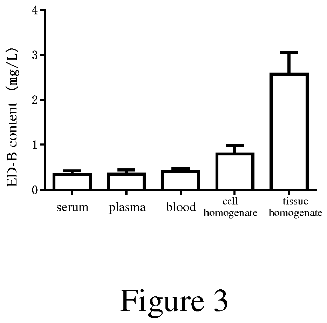

The results showed that (1) the ELISA according to the invention provides a method for quantitative determination of ED-B content, and (2) the detectable samples include: blood samples including serum, plasma and diluted fresh blood samples, and cell and tissue samples. The results were shown in FIG. 3.

## Claims

1. A method for detecting tissue hyperplasia in a mammal, such as human being, diagnosing the presence or risk of solid tumors, or prognosing solid tumors, comprising:
- detecting the level of ED-B protein in a blood sample, of the mammal, and
- comparing with the level of ED-B protein in a healthy control blood sample,
wherein the increased level of the ED-B protein in the detected sample in comparison to the healthy control is indicative of the presence of tissue hyperplasia or the presence or risk of solid tumors in the mammal, or poor prognosis,
wherein the level of the ED-B protein in the biological sample is detected by means of a binding agent specifically detecting the ED-B protein, wherein the ED-B protein comprises: (a) SEQ ID NO:1, or (b) a sequence having at least 80%, 85%, 90%, 95%, 96%, 97%, 98%, 99% or more sequence identity with the sequence of (a), and
wherein the binding agent specifically detecting the ED-B protein is an antibody to the ED-B protein, such as a monoclonal or polyclonal antibody.

2. The method according to claim 1, wherein the blood sample is a whole blood sample, plasma sample or serum sample.

3. The method according to claim 1 or 2, wherein the tissue hyperplasia is breast hyperplasia, and/or the solid tumor is benign or malignant tumor, such as squamous cell carcinoma, adenocarcinoma or sarcoma.

4. The method according to any one of claims 1-3, wherein the solid tumor is selected from the group consisting of nasopharyngeal cancer, head and neck cancer, liver cancer, biliary tract cancer, gallbladder cancer, colon cancer, duodenal cancer, lung cancer, bladder cancer, cervical cancer, ovarian cancer, endometrial cancer, breast cancer, melanoma, pancreatic cancer, renal cancer, prostate cancer and upper gastrointestinal cancer such as esophageal, cardia, laryngeal or gastric cancer, preferably selected from nasopharyngeal carcinoma, esophagus cancer, gastric cancer, lung cancer and pancreatic cancer.

5. The method according to any one of claims 1-4, wherein the solid tumor is an upper gastrointestinal cancer, e.g. selected from esophageal, cardia, laryngeal and gastric cancer.

6. The method according to claim 1, wherein the antibody to the ED-B protein is selected from the group consisting of CGS-1, CGS-2, L19, B5, BC-1 and C6, preferably B5 and/or L19 antibody.

7. The method according to claim 1, wherein the antibody to the ED-B protein comprises at least two selected from the group consisting of CGS-1, CGS-2, L19, B5, BC-1 and C6, preferably B5 and L19 antibodies.

8. The method according to any one of claims 1-7, comprising:
- contacting a first ED-B protein specific binding agent, such as an antibody, with the blood derived sample to be tested, optionally the first ED-B protein specific binding agent is immobilized on a solid support, such as an ELISA plate;
- adding a second ED-B protein specific binding agent, optionally labeled, for example conjugated with an enzyme, and optionally the second ED-B protein specific binding agent and the first ED-B protein specific binding agent bind to different epitopes on the ED-B protein;
- optionally, adding an antibody against the second ED-B protein specific binding agent, optionally labeled, for example conjugated with an enzyme;
and
- detecting the formed complex,
wherein preferably the first and second ED-B protein specific binding agents are antibodies, optionally selected independently from the group consisting of CGS-1, CGS-2, L19, B5, BC-1 and C6, such as B5 and L19.

9. A kit for detecting ED-B protein in a blood sample, of a mammal such as human being, by the method according to any one of claims 1-8, wherein the kit comprises:
i) the binding agent specifically detecting the ED-B protein in the blood sample, of the mammal, as defined in any one of claims 1-7; and,
ii) an anticoagulant,
wherein the level of the ED-B protein in the biological sample is detected by means of a binding agent specifically detecting the ED-B protein, wherein the ED-B protein comprises: (a) SEQ ID NO:1, or (b) a sequence having at least 80%, 85%, 90%, 95%, 96%, 97%, 98%, 99% or more sequence identity with the sequence of (a), and
wherein the binding agent specifically detecting the ED-B protein is an antibody to the ED-B protein, such as a monoclonal or polyclonal antibody.

10. The kit according to claim 9, wherein the binding agent is an ED-B protein specific antibody, optionally labeled and/or monoclonal or polyclonal antibody, for example, selected from the group consisting of CGS-1, CGS-2, L19, B5, BC-1 and C6.

11. The kit according to claim 10, comprising at least two ED-B protein specific antibodies, such as B5 and L19, optionally labeled, and optionally one of them is immobilized on a solid support, such as an ELISA plate.

12. The kit according to any one of claims 9-11, wherein the anticoagulant is heparin, hirudin, sodium citrate or ethylenediaminetetraacetic acid (EDTA), preferably sodium citrate;
and wherein the kit optionally further comprises an ED-B protein standard, such as FN(7B8) encoded by SEQ ID NO. 3.

13. Use of a binding agent specifically detecting ED-B protein, or a kit as defined in any one of claims 9-12, for the detection of ED-B protein in a blood sample, of a mammal such as human being,
wherein the level of the ED-B protein in the biological sample is detected by means of a binding agent specifically detecting the ED-B protein, wherein the ED-B protein comprises: (a) SEQ ID NO:1, or (b) a sequence having at least 80%, 85%, 90%, 95%, 96%, 97%, 98%, 99% or more sequence identity with the sequence of (a), and
wherein the binding agent specifically detecting the ED-B protein is an antibody to the ED-B protein, such as a monoclonal or polyclonal antibody.

14. The use according to claim 13, wherein the binding agent is as defined in any one of claims 1-7.

## Patentansprüche

1. Verfahren zum Nachweisen von Gewebehyperplasie bei einem Säugetier, wie zum Beispiel einem Menschen, Diagnostizieren der Präsenz oder des Risikos von soliden Tumoren oder zur Prognose solider Tumore, das Folgendes umfasst:
- Nachweisen des ED-B-Proteinspiegels in einer Blutprobe des Säugetiers, und
- Vergleichen mit dem ED-B-Proteinspiegel in einer gesunden Kontrollblutprobe,
wobei der erhöhte Spiegel des ED-B-Proteins in der nachgewiesenen Probe im Vergleich zu der gesunden Kontrolle bezeichnend für die Präsenz von Gewebehyperplasie oder die Präsenz oder das Risiko von soliden Tumoren in dem Säugetier oder eine schlechte Prognose ist,
wobei der Spiegel des ED-B-Proteins in der biologischen Probe mittels eines Bindemittels nachgewiesen wird, das das ED-B-Protein spezifisch nachweist, wobei das ED-B-Protein Folgendes umfasst: (a) SEQ ID NO:1 oder (b) eine Sequenz mit mindestens 80 %, 85 %, 90 %, 95 %, 96 %, 97 %, 98 %, 99 % oder mehr Sequenzidentität mit der Sequenz von (a), und
wobei das Bindemittel, das das ED-B-Protein spezifisch nachweist, ein Antikörper gegen das ED-B-Protein ist, wie zum Beispiel ein monoklonaler oder polyklonaler Antikörper.

2. Verfahren nach Anspruch 1, wobei die Blutprobe eine Vollblutprobe, eine Plasmaprobe oder eine Serumprobe ist.

3. Verfahren nach Anspruch 1 oder 2, wobei die Gewebehyperplasie Brusthyperplasie ist und/oder der solide Tumor ein gutartiger oder bösartiger Tumor ist, wie zum Beispiel ein Plattenepithelkarzinom, ein Adenokarzinom oder ein Sarkom.

4. Verfahren nach einem der Ansprüche 1-3, wobei der solide Tumor aus der Gruppe ausgewählt ist, die aus Folgendem besteht: Nasopharynxkrebs, Kopf- und Halskrebs, Leberkrebs, Gallengangkrebs, Gallenblasenkrebs, Dickdarmkrebs, Zwölffingerdarmkrebs, Lungenkrebs, Blasenkrebs, Gebärmutterhalskrebs, Eierstockkrebs, Gebärmutterkrebs, Brustkrebs, Melanom, Bauchspeicheldrüsenkrebs, Nierenkrebs, Prostatakrebs und Krebs des oberen Gastrointestinaltrakts wie zum Beispiel Speiseröhren-, Kardia-, Kehlkopf- oder Magenkrebs, vorzugsweise ausgewählt aus Nasopharynxkarzinom, Speiseröhrenkrebs, Magenkrebs, Lungenkrebs und Bauchspeicheldrüsenkrebs.

5. Verfahren nach einem der Ansprüche 1-4, wobei der solide Tumor ein Krebs des oberen Gastrointestinaltrakts ist, z.B. ausgewählt aus Speiseröhren-, Kardia-, Kehlkopf- und Magenkrebs.

6. Verfahren nach Anspruch 1, wobei der Antikörper gegen das ED-B-Protein aus der Gruppe ausgewählt ist, die aus CGS-1, CGS-2, L19, B5, BC-1 und C6, vorzugsweise B5 und/oder L19 Antikörpern besteht.

7. Verfahren nach Anspruch 1, wobei der Antikörper gegen das ED-B-Protein mindestens zwei umfasst, die aus der Gruppe ausgewählt sind, die aus CGS-1, CGS-2, L19, B5, BC-1 und C6, vorzugsweise B5 und L19 Antikörpern besteht.

8. Verfahren nach einem der Ansprüche 1-7, das Folgendes umfasst:
- Inkontaktbringen eines ersten ED-B-Protein-spezifischen Bindemittels, wie zum Beispiel eines Antikörpers, mit der zu testenden Blutprobe, wobei gegebenenfalls das erste ED-B-Protein-spezifische Bindemittel auf einem soliden Träger, wie zum Beispiel einer ELISA-Platte, immobilisiert ist;
- Hinzufügen eines zweiten ED-B-Protein-spezifischen Bindemittels, das gegebenenfalls markiert, zum Beispiel mit einem Enzym konjugiert ist, und wobei gegebenenfalls das zweite ED-B-Protein-spezifische Bindemittel und das erste ED-B-Protein-spezifische Bindemittel an unterschiedliche Epitope auf dem ED-B-Protein binden;
- gegebenenfalls Hinzufügen eines Antikörpers gegen das zweite ED-B-Protein-spezifische Bindemittel, das gegebenenfalls markiert, zum Beispiel mit einem Enzym konjugiert ist;
und
- Nachweisen des gebildeten Komplexes,
wobei vorzugsweise das erste und das zweite ED-B-Protein-spezifische Bindemittel Antikörper sind, die gegebenenfalls unabhängig aus der Gruppe ausgewählt sind, die aus CGS-1, CGS-2, L19, B5, BC-1 und C6 besteht, wie zum Beispiel B5 und L19.

9. Kit zum Nachweisen von ED-B-Protein in einer Blutprobe eines Säugetiers, wie zum Beispiel eines Menschen, durch das Verfahren nach einem der Ansprüche 1-8, wobei das Kit Folgendes umfasst:
i) das Bindemittel, das das ED-B-Protein in der Blutprobe des Säugetiers nach einem der Ansprüche 1-7 spezifisch nachweist; und,
ii) ein Antikoagulans,
wobei der Spiegel des ED-B-Proteins in der biologischen Probe mittels eines Bindemittels nachgewiesen wird, das das ED-B-Protein spezifisch nachweist, wobei das ED-B-Protein Folgendes umfasst: (a) SEQ ID NO:1 oder (b) eine Sequenz mit mindestens 80 %, 85 %, 90 %, 95 %, 96 %, 97 %, 98 %, 99 % oder mehr Sequenzidentität mit der Sequenz von (a), und
wobei das Bindemittel, das das ED-B-Protein spezifisch nachweist, ein Antikörper gegen das ED-B-Protein ist, wie zum Beispiel ein monoklonaler oder polyklonaler Antikörper.

10. Kit nach Anspruch 9, wobei das Bindemittel ein ED-B-Protein-spezifischer Antikörper ist, gegebenenfalls ein markierter und/oder monoklonaler oder polyklonaler Antikörper, zum Beispiel ausgewählt aus der Gruppe, die aus CGS-1, CGS-2, L19, B5, BC-1 und C6 besteht.

11. Kit nach Anspruch 10, das mindestens zwei ED-B-Protein-spezifische Antikörper umfasst, wie zum Beispiel B5 und L19, gegebenenfalls markiert und gegebenenfalls ist einer von ihnen auf einem soliden Träger immobilisiert, wie zum Beispiel einer ELISA-Platte.

12. Kit nach einem der Ansprüche 9-11, wobei das Antikoagulans Heparin, Hirudin, Natriumcitrat oder Ethylendiamintetraessigsäure (EDTA) ist, vorzugsweise Natriumcitrat;
und wobei das Kit gegebenenfalls ferner einen ED-B-Proteinstandard umfasst, wie zum Beispiel FN(7B8), codiert durch SEQ ID NO. 3.

13. Verwendung eines Bindemittels, das das ED-B-Protein spezifisch nachweist, oder eines Kits nach einem der Ansprüche 9-12 für den Nachweis von ED-B-Protein in einer Blutprobe eines Säugetiers, wie zum Beispiel eines Menschen,
wobei der Spiegel des ED-B-Proteins in der biologischen Probe mittels eines Bindemittels nachgewiesen wird, das das ED-B-Protein spezifisch nachweist, wobei das ED-B-Protein Folgendes umfasst: (a) SEQ ID NO:1 oder (b) eine Sequenz mit mindestens 80 %, 85 %, 90 %, 95 %, 96 %, 97 %, 98 %, 99 % oder mehr Sequenzidentität mit der Sequenz von (a), und
wobei das Bindemittel, das das ED-B-Protein spezifisch nachweist, ein Antikörper gegen das ED-B-Protein ist, wie zum Beispiel ein monoklonaler oder polyklonaler Antikörper.

14. Verwendung nach Anspruch 13, wobei das Bindemittel nach einem der Ansprüche 1-7 ist.

## Revendications

1. Procédé pour détecter une hyperplasie tissulaire chez un mammifère, tel qu'un être humain, diagnostiquer la présence ou le risque de tumeurs solides ou établir le pronostic de tumeurs solides, comprenant :
- détecter le taux de protéine ED-B dans un échantillon de sang, du mammifère, et
- le comparer avec le taux de protéine ED-B dans un échantillon de sang témoin sain,
où le taux accru de protéine ED-B dans l'échantillon détecté par rapport au témoin sain est indicatif de la présence d'une hyperplasie tissulaire ou de la présence ou du risque de tumeurs solides chez le mammifère, ou d'un mauvais pronostic,
où le taux de protéine ED-B dans l'échantillon biologique est détecté au moyen d'un agent de liaison détectant spécifiquement la protéine ED-B, où la protéine ED-B comprend : (a) SEQ ID NO : 1, ou (b) une séquence ayant au moins 80 %, 85 %, 90 %, 95 %, 96 %, 97 %, 98 %, 99 % ou plus d'identité de séquence avec la séquence de (a), et
où l'agent de liaison détectant spécifiquement la protéine ED-B est un anticorps contre la protéine ED-B, tel qu'un anticorps monoclonal ou polyclonal.

2. Procédé selon la revendication 1, où l'échantillon de sang est un échantillon de sang total, un échantillon de plasma ou un échantillon de sérum.

3. Procédé selon la revendication 1 ou 2, où l'hyperplasie tissulaire est une hyperplasie mammaire, et/ou la tumeur solide est une tumeur bénigne ou maligne, telle qu'un carcinome épidermoïde, un adénocarcinome ou un sarcome.

4. Procédé selon l'une quelconque des revendications 1 à 3, où la tumeur solide est choisie dans le groupe consistant en cancer du nasopharynx, cancer de la tête et du cou, cancer du foie, cancer des voies biliaires, cancer de la vésicule biliaire, cancer du côlon, cancer du duodénum, cancer du poumon, cancer de la vessie, cancer du col de l'utérus, cancer de l'ovaire, cancer de l'endomètre, cancer du sein, mélanome, cancer du pancréas, cancer du rein, cancer de la prostate et cancer des voies digestives supérieures tel que le cancer de l'oesophage, du cardia, du larynx ou de l'estomac, de préférence choisi parmi le carcinome du nasopharynx, le cancer de l'oesophage, le cancer de l'estomac, le cancer du poumon et le cancer du pancréas.

5. Procédé selon l'une quelconque des revendications 1 à 4, où la tumeur solide est un cancer des voies digestives supérieures, par exemple sélectionné parmi le cancer de l'oesophage, du cardia, du larynx et de l'estomac.

6. Procédé selon la revendication 1, où l'anticorps contre la protéine ED-B est choisi dans le groupe constitué par les anticorps CGS-1, CGS-2, L19, B5, BC-1 et C6, de préférence B5 et/ou L19.

7. Procédé selon la revendication 1, où l'anticorps contre la protéine ED-B comprend au moins deux anticorps sélectionnés dans le groupe consistant en CGS-1, CGS-2, L19, B5, BC-1 et C6, de préférence B5 et L19.

8. Procédé selon l'une quelconque des revendications 1 à 7, comprenant :
- mettre en contact un premier agent de liaison spécifique à la protéine ED-B, tel qu'un anticorps, avec l'échantillon dérivé du sang à tester, facultativement le premier agent de liaison spécifique à la protéine ED-B est immobilisé sur un support solide, tel qu'une plaque ELISA ;
- ajouter un deuxième agent de liaison spécifique à la protéine ED-B, facultativement marqué, par exemple conjugué avec une enzyme, et facultativement le deuxième agent de liaison spécifique à la protéine ED-B et le premier agent de liaison spécifique à la protéine ED-B se lient à des épitopes différents sur la protéine ED-B ;
- facultativement, ajouter un anticorps contre le deuxième agent de liaison spécifique à la protéine ED-B, facultativement marqué, par exemple conjugué à une enzyme ;
et
- détecter le complexe formé,
où, de préférence, le premier et le deuxième agents de liaison spécifique à la protéine ED-B sont des anticorps, facultativement sélectionnés indépendamment dans le groupe consistant en CGS-1, CGS-2, L19, B5, BC-1 et C6, tels que B5 et L19.

9. Kit pour détecter la protéine ED-B dans un échantillon de sang, d'un mammifère tel qu'un être humain, au moyen du procédé selon l'une quelconque des revendications 1 à 8, où le kit comprend :
i) l'agent de liaison détectant spécifiquement la protéine ED-B dans l'échantillon de sang, du mammifère, tel que défini dans l'une quelconque des revendications 1 à 7 ; et,
ii) un anticoagulant,
où le taux de la protéine ED-B dans l'échantillon biologique est détecté au moyen d'un agent de liaison détectant spécifiquement la protéine ED-B, où la protéine ED-B comprend : (a) SEQ ID NO : 1, ou (b) une séquence ayant au moins 80 %, 85 %, 90 %, 95 %, 96 %, 97 %, 98 %, 99 % ou plus d'identité de séquence avec la séquence de (a), et
où l'agent de liaison détectant spécifiquement la protéine ED-B est un anticorps contre la protéine ED-B, tel qu'un anticorps monoclonal ou polyclonal.

10. Kit selon la revendication 9, où l'agent de liaison est un anticorps spécifique à la protéine ED-B, facultativement marqué et/ou un anticorps monoclonal ou polyclonal, par exemple, choisi dans le groupe consistant en CGS-1, CGS-2, L19, B5, BC-1 et C6.

11. Kit selon la revendication 10, comprenant au moins deux anticorps spécifiques à la protéine ED-B, tels que B5 et L19, facultativement marqués, et facultativement l'un d'entre eux est immobilisé sur un support solide, tel qu'une plaque ELISA.

12. Kit selon l'une quelconque des revendications 9 à 11, où l'anticoagulant est l'héparine, l'hirudine, le citrate de sodium ou l'acide éthylènediaminetétraacétique (EDTA), de préférence le citrate de sodium ; et
où le kit comprend facultativement en outre un étalon de protéine ED-B, tel que FN(7B8) codé par SEQ ID NO. 3.

13. Utilisation d'un agent de liaison détectant spécifiquement la protéine ED-B, ou d'un kit tel que défini dans l'une quelconque des revendications 9 à 12, pour la détection de la protéine ED-B dans un échantillon de sang, d'un mammifère tel qu'un être humain,
où le taux de la protéine ED-B dans l'échantillon biologique est détecté au moyen d'un agent de liaison détectant spécifiquement la protéine ED-B, où la protéine ED-B comprend : (a) SEQ ID NO : 1, ou (b) une séquence ayant au moins 80 %, 85 %, 90 %, 95 %, 96 %, 97 %, 98 %, 99 % ou plus d'identité de séquence avec la séquence de (a), et
où l'agent de liaison détectant spécifiquement la protéine ED-B est un anticorps contre la protéine ED-B, tel qu'un anticorps monoclonal ou polyclonal.

14. Utilisation selon la revendication 13, où l'agent de liaison est tel que défini dans l'une quelconque des revendications 1 à 7.
